# EUROPEAN PATENT SPECIFICATION

(11) **EP 2 931 060 B1**
(45) Date of publication and mention of the grant of the patent: **06.09.2017**
(21) Application number: 12809491.9
(22) Date of filing: 14.12.2012
(51) Int. Cl.: A23K 10/30, A23K 10/20, A23K 50/40

(54) **ANTI-AGING FOODS FOR COMPANION ANIMALS**
ANTI-AGING-NAHRUNGSMITTEL FÜR HAUSTIERE
ALIMENTS ANTI-VIEILLISSEMENT POUR ANIMAUX DE COMPAGNIE

(43) Date of publication of application: 21.10.2015
(73) Proprietor: Hill's Pet Nutrition, Inc., Topeka, KS 66603 (US)
(72) Inventor: JEWELL, Dennis, Lawrence, Kansas 66049 (US); BROCKMAN, Jeffrey, Lawrence, KS 66047 (US)
(74) Representative: Wichmann, Hendrik
(86) International application number: PCT/US2012/069611
(87) International publication number: WO 2014/092717

(56) References cited:
- WO-A1-2011/011472
- WO-A1-2012/100991
- US-A1- 2001 018 067

## Description

### BACKGROUND OF THE INVENTION

Aging has been defined as an increase in the probability of death. However, aging, *per se,* is a natural process, not a medical condition. Although aging is by definition, accompanied by an eventual physiological decline, it can be readily observed that the rate of progression and the consequences of this process are not necessarily uniform between animal genera and species, and even between and among individual members of an animal species. The rate of physiological decline and the overall health of an individual aging companion animal appear to reflect the influence of not only ill-defined genetic determinants but also environmental factors.

Animals may be afflicted with one or more conditions that appear to be age-related and that individually and collectively affect the overall health and longevity of the individual animal as it ages. It has been suggested that animals do not die of "healthy aging" but rather they die from these age-related diseases. Illustrative age-related conditions of felines include, *inter alia,* deterioration of muscle protein, deterioration of cartilage, accumulation of body fat and/or a decrease in lean body mass, deterioration in kidney health, untoward, inappropriate or excessive inflammatory responses, gastrointestinal health conditions and oxidative damage.

Accordingly, there is a need for compositions and methods for treatment and amelioration of those age-associated conditions that would support "healthy aging," providing the treated animal with a healthier and/or longer life.

US2001018067 discloses a process for limiting weight gain in cats. The process includes feeding the cat a pet food composition that includes a source of protein, a source of fat, and a source of carbohydrates from a grain source that excludes rice.

WO2012100991 discloses microorganisms or fragments thereof as sensorically neutral oral care agents, particularly for prevention of dental calculus, as anti-caries agents and/or anti-oral malodor agents.

WO2011011472 discloses a nutrition system for a companion animal that includes a plurality of pet food packets, wherein an amount of pet food corresponding to a single meal for a companion animal may be selected and wherein the amount of pet food is formulated to alleviate or manage a disorder in a companion animal.

### BRIEF SUMMARY OF THE INVENTION

The present invention provides a composition according to claim 1. The present disclosure generally relates to compositions and methods for the prevention and treatment of age-related conditions in companion animals in need thereof.

The methods for treating or preventing an age-related condition in an animal in need thereof, comprise administering to the animal an effective amount of a composition comprising at least one of a protein source, a carbohydrate source, and a vegetable source, or a combination of two or more thereof. In this method, the protein source is selected from the group consisting of chicken, "pea protein concentrate," corn gluten meal, and combinations thereof; the carbohydrate source is selected from the group consisting of brown rice, oat groats, and combinations thereof, and the vegetable source is selected from the group consisting of beet pulp, tomato pomace, broccoli powder, and combinations thereof.

In one embodiment of the method, the age-related condition is one of inappropriate immune response and/or inflammation, gastrointestinal disease, defective and/or suboptimal digestive function, increased body fat, decreased lean body mass, decreased kidney health oxidative damage, or a combination of two or more thereof.

In another embodiment, the animal is a companion animal. In one aspect of this embodiment, the companion animal is a canine, while in another, the companion animal is a feline.

In other embodiments of the method, the administered composition is a nutritionally complete diet for an adult companion animal. In one aspect of this embodiment, the administered composition is a nutritionally complete diet for an adult canine while, in another, the administered composition is a nutritionally complete diet for an adult feline.

In another embodiment of the method, the administered composition is a nutritionally complete diet for an aged companion animal. In one aspect of this embodiment, the administered composition is a nutritionally complete diet for an aged canine while, in another, the administered composition is a nutritionally complete diet for an aged feline.

In particular embodiments, practice of the method results in an improvement in at least one of lean body mass, gut health, digestive health, kidney health, immune system function, inflammation, oxidative defenses, or a combination of two or more thereof, in the treated animal. In specific aspects, the method of prevention or treatment results in at least one of decreased adiposity, increased lean body mass, decreased levels of 8-hydroxyguanosine, increased circulating levels of L-camitine, decreased levels of circulating oxidized glutathione, decreased levels of circulating *p*-cresol sulfate accumulation, decreased levels of circulating 3-inoxyl sulfate, or a combination of two or more thereof, in the treated animal.

The present disclosure also encompasses compositions for treating or preventing an age-related condition in an animal in need thereof, where the composition comprises a protein source, a carbohydrate source, and a vegetable source, or a combination of two or more thereof. In this embodiment, the protein source is selected from the group consisting of chicken, "pea protein concentrate," corn gluten meal, and combinations thereof, the carbohydrate source is selected from the group consisting of brown rice, oat groats, and combinations thereof, and the vegetable source is selected from the group consisting of beet pulp, tomato pomace, broccoli powder, and combinations thereof.

In certain embodiments, the compositions of the present disclosure are nutritionally complete diets for an adult companion animal. In particular aspects of this embodiment, the composition is a nutritionally complete diet for a canine while in another aspect the composition is a nutritionally complete diet for a feline.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

The present disclosure is directed to compositions and methods for treating or preventing an age-related condition in a companion animal in need thereof. Compositions of the disclosure, effective amounts of which are administered in the methods of the disclosure, include at least one of a protein source, a carbohydrate source, and a vegetable source, or a combination of two or more thereof, wherein the protein source is selected from the group consisting of chicken, pea protein concentrate, corn gluten meal, and combinations thereof, wherein the carbohydrate source is selected from the group consisting brown rice, oat groats, and combinations thereof, and wherein the vegetable source is selected from the group consisting of beet pulp, tomato pomace, broccoli powder and combinations thereof.

### Definitions

Unless defined otherwise, all technical and scientific terms used herein have the same meanings as commonly understood by one of ordinary skill in the art to which this invention belongs.

As used herein and in the appended claims, the singular forms "a", "an", and "the" include plural reference unless the context clearly dictates otherwise.

As used herein, the term "animal" refers to companion animals. In one specific aspect, the animal is a dog, while in another, the animal is a cat.

According to the present disclosure, the phrase "animal in need thereof," refers to a companion animal for which treatment, prevention, or control of an age-related condition is indicated. Such animals include those known to be currently exhibiting the symptoms of or known to be afflicted with an age-related condition, as well as those known to be or expected to be at risk of developing an age-related condition. Animals known to or expected to be at risk of developing an age-related condition include, for example, members of species or subgroups thereof, that are known or inferred to carry one or more genetic determinants associated with the very occurrence or that earlier onset or rate of development of one or more age-related conditions, as compared to members of closely-related genera, species and/or subgroups thereof.

The term "preventing," as used herein, means e.g., to completely or almost completely stop, as well as to substantially reduce development or progression of an age-related condition by the animal in need thereof.

The term "treating," as used herein, means to cure, inhibit, arrest the development, relieve the symptoms or effects of, or to ameliorate, or cause the reduction in the symptoms or effects of an age-related condition in an animal in need of methods disclosed herein. Accordingly, it should be recognized that although the terms "preventing," treating," and "controlling," are distinguishable from one another, there can be overlap amongst these terms.

The term "companion animal" used in the present disclosure includes any non-human animal suitable for being kept as a pet by humans including without limitation, a dog, a cat, rabbit and a rodent. Specific embodiments of the present disclosure are formulations and methods of treatment for dogs and/or cats. In one specific aspect, the present disclosure is directed to formulations and methods of treatment of cats.

The term "dog" includes those dogs which are companion animals such as *Canis familiaris,* working dogs and the like. The term dog is synonymous with the term canine.

The term "cat" includes those cats which are companion animals known as domestic cats or house cats, or *Felis domesticus.* The term cat is synonymous with the term feline.

As used herein, "an amount effective", "an effective amount", and like terms refer to that amount of a material or composition as described herein that may be effective to achieve a particular biological result, *i.e.,* prevention, treatment, or amelioration of an age-related condition. In specific embodiments, administration of an effective amount of a composition of the disclosure will be for a time sufficient to prevent, treat, or ameliorate an age-related condition or one or more effects or manifestations thereof in the animal treated. In a particular embodiment, the method comprises administration and consumption of a composition of the disclosure for a period of time sufficient to result in prevention, treatment, or amelioration of an age-related condition or one or more effects thereof in the animal treated to a level acceptable to the owner of a non-human animal in need of the methods of prevention and treatment disclosed herein. An effective amount may be based on several factors, including an animal's ideal weight, the age, gender, and activity of the animal, the metabolizable energy of the composition, and the frequency of feeding the compositions of the present disclosure, e.g., once, twice, or three times daily, and other compositions fed to the animal.

A "food," "food composition," or "pet food composition" can, in some embodiments of the disclosure, be a nutritionally complete diet for the intended recipient animal *(e.g.,* a companion animal, such as a domestic cat or domestic dog).

As used herein, an "ingredient" refers to any component of a composition.

The term "nutrient" refers to a substance that provides nourishment. In some cases an ingredient may comprise more than one "nutrient," for example, a composition may comprise corn comprising important nutrients including both protein and carbohydrate.

As contemplated herein, the compositions of the present invention are meant to encompass, but not be limited to, nutritionally-complete and balanced animal food compositions. A "nutritionally complete diet" is a diet that includes sufficient nutrients for maintenance of normal health of a healthy animal on the diet. Nutritionally complete and balanced pet food compositions, *e.g.,* for companion felines and canines, are familiar to one of skill in the art. For example substances such as nutrients and ingredients suitable for nutritionally complete and balanced animal feed compositions, and recommended amounts thereof, may be found for example, in the Official Publication of the Association of American Feed Control Officials, Inc. (AAFCO), Atlanta, GA, (2012).

As used herein, the term "supplement(s)" include, but are not limited to, a feed used with another feed to improve nutritive balance or performance of the total diet for an animal. Supplements include, but are not limited to, compositions that are fed undiluted as a supplement to other feeds, offered free choice with other parts of an animal's ration that are separately available, or diluted and mixed with an animal's regular feed to produce a complete feed. The AAFCO guidelines, for example, contain a discussion relating to supplements in the Official Publication of the Association of American Feed Control Officials, Inc. (AAFCO), Atlanta, GA (2012). Supplements may be in various forms including, for example, powders, liquids, syrups, pills, encapsulated compositions and the like.

"Bioactive dietary components" (BDCs) may include amino acids, simple and complex sugars, vitamins, cofactors, antioxidants, omega-3 fatty acids, various botanical preparations, *etc.* Certain materials can be considered BDCs that may contain one or more bioactive components, that, whether known or unknown, may additively or synergistically contribute to the bioactive effect of the material.

Aging is the natural process in animals that is characterized by progressive degenerative changes in tissue organization and function that increase the probability of mortality. Associated with the natural aging process are age-related condition, the age of onset, development, and rate of progression of which have both genetic and environmental components. Accordingly, age-related conditions include those conditions that observed in and associated with older, aging animals, at least as compared to younger animals of the same genus and species. Illustrative, non-limiting examples of age-related conditions include, deterioration of muscle protein, deterioration of cartilage, accumulation of body fat and/or a decrease in lean body mass, deterioration in kidney health, untoward, inappropriate or excessive inflammatory responses, gastrointestinal health conditions and oxidative damage. The age of onset, development, and rate of progression of such age-related conditions, may vary substantially between and among members of the same species.

### Compositions and Formulations

Application of the methodology outlined above has identified bioactive dietary components that have been combined to provide compositions, foods, and diets that provide significant anti-aging benefits to animals, e.g., to companion animals such as canines and felines, and, in a particular embodiment, to domesticated cats. When administered to animals in need thereof, these compositions, foods, and diets are observed to improve body composition, improve renal function, decrease circulating levels of toxic metabolites, and improve the oxidative state of the treated animals. These studies identified three protein sources (chicken, pea protein concentrate, and corn gluten meal) as useful in compositions administered in the presently-described methods for the prevention and/or treatment of age-related conditions in animals, e.g., canines and, in particular, to felines. These studies also identified two carbohydrate sources (brown rice and oat groats) as well as specific vegetables (beet pulp, tomato pomace, and broccoli powder) useful in the compositions administered in the presently disclosed methods.

Accordingly, compositions of the disclosure, which are administered in the methods described herein for prevention and/or treatment of an age-related condition in an animal in need thereof, comprise a protein source, a carbohydrate source, and a vegetable source, or a combination of two or more thereof. The protein source is selected from the group consisting of chicken, pea protein concentrate, corn gluten meal, and combinations thereof, while the carbohydrate source is selected from the group consisting of brown rice and oat groats, and combinations thereof. The vegetable source is selected from the group consisting of beet pulp, tomato pomace, broccoli powder and combinations thereof.

Particular embodiments of the present disclosure are directed to mixtures of these bioactive dietary components as ingredients to provide compositions and formulations that, when administered to an animal in need thereof, impart specific anti-aging effects.

In one aspect of these embodiments, the compositions comprise chicken in an amount from 5% to 25% based on the total weight of the composition on a dry matter basis.

In another aspect, the compositions comprise "pea protein concentrate" in an amount from 10% to 45% based on the total weight of the composition on a dry matter basis.

In another aspect, the compositions comprise corn gluten meal in an amount from 10% to 45% based on the total weight of the composition on a dry matter basis.

In a further aspect, the compositions comprise brown rice in an amount from 8% to 40% based on the total weight of the composition on a dry matter basis.

In another aspect, the compositions comprise oat groats in an amount from 8% to 40% based on the total weight of the composition on a dry matter basis.

In still another aspect of these embodiments, the compositions comprise one or more of the indicated vegetables, beet pulp, tomato pomace, and broccoli powder, in an amount from 1.9% to 10% based on the total weight of the composition on a dry matter basis.

In certain embodiments, compositions of the present disclosure comprise chicken in an amount of 5%, 7.5%, 10%, 12.5%, 15%, 17.5%, 20%, 22.5% or 25% based on the total weight of the composition on a dry matter basis. In particular aspects of these embodiments, compositions of the disclosure may comprise a dry weight of chicken within a range defined by any two of these values as endpoints.

In certain embodiments, compositions of the present disclosure comprise pea protein concentration in an amount of 10%, 15%, 20%, 25%, 30%, 35%, 40%, or 45% based on the total weight of the composition on a dry matter basis. In particular aspects of these embodiments, compositions of the disclosure may comprise a dry weight of pea protein concentrate within a range defined by any two of these values as endpoints.

In certain embodiments, compositions of the present disclosure comprise corn gluten meal in an amount of 10%, 15%, 20%, 25%, 30%, 35%, 40%, or 45% based on the total weight of the composition on a dry matter basis. In particular aspects of these embodiments, compositions of the disclosure may comprise a dry weight of corn gluten meal within a range defined by any two of these values as endpoints.

In certain embodiments, compositions of the present disclosure comprise brown rice in an amount of 8%, 10%, 15%, 20%, 25%, 30%, 35%, or 40% based on the total weight of the composition on a dry matter basis. In particular aspects of these embodiments, compositions of the disclosure may comprise a dry weight of brown rice within a range defined by any two of these values as endpoints.

In certain embodiments, compositions of the disclosure comprise oat groats in an amount of 8%, 10%, 15%, 20%, 25%, 30%, 35%, or 40% based on the total weight of the composition on a dry matter basis. In particular aspects of these embodiments, compositions of the disclosure may comprise a dry weight of oat groats within a range defined by any two of these values as endpoints.

In still another aspect of these embodiments, compositions of the disclosure comprise one or more of the indicated vegetables, beet pulp, tomato pomace, and broccoli powder, in an amount of 1.9%, 2%, 3% 4%, 5%, 6%, 7%, 8%, 9% or 10% based on the total weight of the composition on a dry matter basis. In particular aspects of these embodiments, compositions of the disclosure may comprise a dry weight of vegetables, comprising one or more of beet pulp, tomato pomace, and broccoli powder, within a range defined by any two of these values as endpoints.

In other aspects of this embodiment, the food product is a nutritionally complete diet for an adult companion animal, e.g., an adult canine or an adult feline companion animal. In a specific aspect, the food product is a nutritionally complete diet formulated for an adult companion feline.

In other aspects of this embodiment, the food product is a nutritionally complete diet for an aged companion animal, e.g., an aged canine or an adult feline companion animal. In a specific aspect, the food product is a nutritionally complete diet formulated for an aged companion feline.

Compositions of the present disclosure include food compositions that may comprise from 10% to 75% protein based on the total weight of the composition on a dry matter basis, from 5% to 50% fat based on the total weight of the composition on a dry matter basis, and from 8% to 75% carbohydrate based on the total weight of the composition on a dry matter basis, wherein the food composition is suitable for consumption by an animal, and wherein the composition is effective for prevention and/or treatment of an age-related condition in an animal in need thereof.

The compositions of the disclosure, which are administered in the methods of the disclosure, may be formulated as an animal food composition that, in certain embodiments, is a nutritionally-balanced and/or nutritionally-complete animal food product or diet. In other embodiments, the composition is formulated and prepared as a nutritional supplement, a treat, or a toy.

For example, a nutritionally complete and balanced cat food composition of the present disclosure may comprise: from 10 to 90%, from 15% to 75%, from 20% to 60% protein, and from 25% to 50% by weight of protein; from 8% to 75%, from 10% to 60%, and from 5% to 50% by weight of carbohydrate; from 2% to 60%, from 5% to 50%, and from 10% to 35% by weight of fat. The compositions may further contain from 0 to 15%, or from 2% to 8%, by weight of vitamins *(e.g.,* vitamin E) and minerals, antioxidants, and other nutrients, *e.g.* amino acids (*e.g.,* methionine, DL-methionine, and L-methionine), which support the nutritional needs of the animal.

For example, a nutritionally complete and balanced dog food composition of the present disclosure may comprise: from 4 to 90%, from 5% to 75%, from 10% to 60% protein, and from 15% to 50% by weight of protein; from 0% to 90%, from 2% to 80%, from 5% to 75%, and from 10% to 50% by weight of carbohydrate; from 2% to 60%, from 5% to 50%, and from 10% to 35% by weight of fat. The compositions may further contain from 0 to 15%, or from 2% to 8%, by weight of vitamins and minerals, antioxidants, and other nutrients which support the nutritional needs of the animal.

Sources of proteins, carbohydrates, fats, vitamins, minerals, balancing agents, and the like, suitable for inclusion in the compositions of the disclosure, and particularly in the food products of the disclosure to be administered in the claimed methods, may be selected from among those conventional materials known to those of ordinary skill in the art.

Proteins useful as ingredients of the food compositions of the present disclosure may, in addition to one or more of chicken, pea protein concentrate, and corn gluten meal, be from any source, including, for example, proteins from animal sources, such as meat protein isolate, whey protein isolate, mixtures thereof, and the like, as well as vegetable sources, such as soy protein isolate, corn, wheat gluten, mixtures thereof, and the like. Additional sources of protein may include one or more of the following: animal proteins, including mammalian, avian protein, reptilian, amphibian, fish, invertebrate proteins and combinations thereof; e.g., from any of cattle, sheep, pig, goat, deer, rabbit, horse, kangaroo, their milk, curds, whey or blood, and internal tissues and organs such as smooth muscle, striate muscle, liver, kidney, intestine or heart; additional avian protein sources encompass turkey, goose, duck, ostrich, quail, pigeon, their eggs and internal tissues and organs such as smooth muscle, striate muscle, liver, kidney, intestine or heart; amphibian sources include frog or salamander; reptilian protein sources include alligator, lizard, turtle and snake; fish protein sources include catfish, herring, salmon, tuna, bluefish, cod, halibut, trout, swordfish and their eggs; and invertebrate protein sources include lobster, crab, clams, mussels or oysters, and combinations thereof.

Carbohydrate components of compositions of the present disclosure may, in addition to one or more of brown rice and oat groats, be from any source, and may enter the food composition as part of another ingredient, such as the protein source. In certain embodiments, carbohydrates useful as ingredients of the food compositions of the present disclosure include polysaccharides (*e.g.,* starches and dextrins) and sugars (*e.g.,* sucrose, lactose, maltose, glucose, and fructose) that are metabolized for energy when hydrolyzed. Examples of additional carbohydrate sources suitable for inclusion in the compositions disclosed herein include, but are not limited to, corn, whole yellow corn, grain sorghum, wheat, barley, and rice.

Fats useful as ingredients of the food compositions of the present disclosure may be from any source, such as but not limited to poultry fat, beef tallow, lard, choice white grease, soybean oil, corn oil, canola oil, sunflower oil, mixtures thereof, and the like. The fat may be incorporated completely within the food composition, deposited on the outside of the food composition, or a mixture of the two methods.

In one embodiment, the composition to be administered in the described methods is formulated and prepared as a supplement. Supplements include, for example, a food product, feed, or pet food, that can be used with another food product, feed, or pet food composition to improve the nutritive balance or performance of the total. Contemplated supplements include compositions that are fed undiluted as a supplement to other feeds or pet foods, offered free choice with other parts of an animal's ration that are separately available, or diluted and mixed with an animal's regular feed or pet food to produce a complete feed or pet food. The AAFCO, for example, has provided a discussion relating to supplements in the Official Publication of the Association of American Feed Control Officials, Inc. (Atlanta, GA 2012). Supplements may be in various forms including, for example, powders, liquids, syrups, pills, encapsulated compositions, *etc.*

In another embodiment, treats of the present disclosure can be prepared by, for example, an extrusion or baking process similar to those described below for dry food to provide
an edible product. Treats include, for example, compositions that are given to an animal to entice the animal to eat during a non-meal time. Treats may be nutritional, wherein the composition comprises one or more nutrients, and may, for example, have a composition as described above for food. Non-nutritional treats encompass any other treats that are non-toxic. Compositions of the disclosure can be coated onto the treat, incorporated into the treat, or both.

In another embodiment, the animal toy is a chewable or consumable toy, that is typically prepared by coating any existing toy with a formulation of the disclosure. Toys therefore include, for example, chewable toys. Contemplated toys for dogs include, for example, artificial bones. In certain embodiments, the composition of the invention can form a coating on the surface of the toy or on the surface of a component of the toy, or it can be incorporated partially or fully throughout the toy, or both. A wide range of suitable toys are currently marketed. *See, e.g.,* U.S. Pat. No. 5,339,771 (and references disclosed in U.S. Pat. No. 5,339,771). *See also, e.g.,* U.S. Pat. No. 5,419,283 (and references disclosed in U.S. Pat. No. 5,419,283). It should be recognized that this invention contemplates both partially consumable toys (*e.g*., toys comprising plastic components) and fully consumable toys *(e.g.,* rawhides and various artificial bones). It should be further recognized that this invention contemplates toys use by companion animals, particularly for use by a dog or by a cat.

### Preparation of the Compositions of the Invention

The compositions of the invention, which are to be administered to animals in need of the methods disclosed herein, may be prepared as food products suitable for consumption by the animals. These food products may be of any consistency or moisture content; *i.e.,* the compositions of the present invention may be moist, semi-moist, or dry food products. "Moist" food products are generally those with a moisture content of from 60% to 90% or greater. "Dry" food products are generally those with a moisture content of from 3% to 11%, and are often manufactured in the form of small pieces or kibbles. "Semi-moist food products generally have a moisture content of from 25% to 35%. The food products of the present invention may also include components of more than one consistency, for example, soft, chewy meat-like particles or pieces as well as kibble having an outer cereal component or coating and an inner "cream" component, *e.g.,* as described in U.S. Patent No. 6,517,877.

In certain embodiments, the food products may be prepared in a canned or wet form using conventional food preparation processes known to those of ordinary skill in the art. Typically, ground animal proteinaceous tissues are mixed with the other ingredients, such as cereal grains, suitable carbohydrate sources, fats, oils, and balancing ingredients, including special purpose additives such as vitamin and mineral mixtures, inorganic salts, cellulose, and the like, and water in an amount sufficient for processing. The ingredients are mixed in a vessel suitable for heating while blending the components. Heating the mixture is carried out using any suitable manner, for example, direct steam injection or using a vessel fitted with a heat exchanger. Following addition of all of the ingredients of the formulation, the mixture is heated to a temperature of from 10 °C to 100 °C (50 °F to 212 °F). Although temperatures outside this range can be used, they may be commercially-impractical without the use of other processing aids. When heated to the appropriate temperature, the material will typically be in the form of thick liquid, which is dispensed into cans. A lid is applied and the container is hermetically sealed. The sealed can is then placed in conventional equipment designed for sterilization of the contents. Sterilization is usually accomplished by heating to temperatures of greater than 230 °C for an appropriate time depending on the temperature used, the nature of the composition, and related factors. The compositions and food products of the present invention can also be added to or combined with food compositions before, during, or after their preparation.

In other embodiments, the food products may be prepared in a dry form using conventional processes known to those of ordinary skill in the art. Typically, dry ingredients, including dried animal protein, plant protein, grains and the like are ground and mixed together. Liquid or moist ingredients, including fats, oils, animal protein, water, and the like are added combined with the dry materials. The specific formulation, order of addition, combination, and methods and equipment used to combine the various ingredients can be selected from those known in the art. For example, in certain embodiments, the resulting mixture is processed into kibbles or similar dry pieces, which are formed using an extrusion process in which the mixture of dry and wet ingredients is subjected to mechanical work at high pressure and temperature, forced through small openings or apertures, and cut off into the kibbles, e.g., with a rotating knife. The resulting kibble can be dried and optionally coated with one or more topical coatings comprising, e.g., flavors, fats, oils, powdered ingredients, and the like. Kibbles may also be prepared from dough by baking, rather than extrusion, in which the dough is placed into a mold before dry-heat processing.

In preparing a composition for use with the methods of the present disclosure, any ingredient generally may be incorporated into the composition during the processing of the formulation, e.g., during and/or after mixing of the other components of the composition. Distribution of these components into the composition can be accomplished by conventional means. In certain embodiments, ground animal and/or poultry proteinaceous tissues are mixed with other ingredients, including nutritional balancing agents, inorganic salts, and may further include cellulose, bulking agents and the like, along with sufficient water for processing.

In particular embodiments, the compositions are formulated so as to be easier to chew. In specific embodiments, the compositions and food products are formulated to address specific nutritional differences between species and breeds of animals, as well as one of more of the attributes of the animal. For example, canine and feline foods, for example, are typically formulated based upon the life stage, age, size, weight, body composition, and breed.

Accordingly, in one specific embodiment, the present disclosure is directed to a method for preparing a food product, the method comprising, admixing suitable sources of protein, fat, carbohydrate, minerals, and vitamins, and processing the mixture to provide the food product, to provide a food product suitable for consumption by a companion animal such a feline or canine companion animal, in which the food product comprises a protein source, a carbohydrate source, a vegetable source, a fruit source, or a combination of two or more thereof, in which at least one of (1) the protein source is selected from the group consisting of chicken, pea protein concentrate, corn gluten meal, and combinations thereof, (2) the carbohydrate source is selected from the group consisting of brown rice, oat groats, and combinations thereof, and (3) the vegetable source is selected from the group consisting of beat pulp, tomato pomace, broccoli powder and combinations thereof.

In one aspect of these embodiments, the composition is a food product that comprises chicken at a level of from 5% to 25% by dry weight.

In another aspect, the composition is a food product that comprises pea protein concentrate at a level of from 10% to 45% by dry weight.

In another aspect, the composition is a food product that comprises corn gluten meal at a level of from 10% to 45% by dry weight.

In a further aspect, the composition is a food product that comprises brown rice at a level of from 8% to 40% by dry weight of the composition.

In another aspect, the composition is a food product that comprises oat groats at a level of from 8% to 40% by dry weight.

In still another aspect of these embodiments, the composition is a food product that comprises one or more of the indicated vegetables, beet pulp, tomato pomace, and broccoli powder, wherein the total amount of this set of vegetables is at a level of from 1.9% to 10% by dry weight.

In other aspects of this embodiment, the food product is a nutritionally complete diet for an adult companion animal, e.g., an adult canine or an adult feline companion animal. In a specific aspect, the food product is a nutritionally complete diet formulated for an adult companion feline. In another specific embodiment, the food product is a nutritionally complete diet formulated for an adult companion canine.

In other aspects of this embodiment, the food product is a nutritionally complete diet for an aged companion animal, e.g., an aged canine or an adult feline companion animal. In a specific aspect, the food product is a nutritionally complete diet formulated for an aged companion feline. In another specific embodiment, the food product is a nutritionally complete diet formulated for an aged companion canine.

The compositions of the present disclosure that are formulated as a nutritionally complete diet meet the needs of a mature adult or an aged animal, such as a companion canine or feline. These nutritionally complete diets that include sufficient nutrients for maintenance of normal health of a healthy animal on the diet. Nutritionally complete and balanced pet food compositions, e.g. for companion canines and felines, are familiar to one of skill in the art. For example substances such as nutrients and ingredients suitable for nutritionally complete and balanced animal feed compositions, and recommended amounts thereof, may be found for example, in the Official Publication of the Association of American Feed Control Officials, Inc. (AAFCO), Atlanta, GA, (2012).

Compositions of the present disclosure include those that, when administered to an animal in need thereof, result in biological effects that offset one or more degradative effects of aging, providing, e.g., decreased adiposity, increased lean body mass, improved kidney health, improved digestive health, reduced inflammation, and reduced oxidative stress.

### Methods of the Disclosure

The present disclosure is further directed to methods for treating or preventing an age-related condition in an animal in need thereof. These methods comprise administering to the animal an effective amount of a composition of the present disclosure that comprises at least one of a protein source, a carbohydrate source, and a vegetable source, or a combination of two or more thereof. A protein source to be formulated in a composition administered in these methods can be selected from the group consisting of chicken, pea protein concentrate, corn gluten meal, and combinations thereof. A carbohydrate source to be formulated in a composition administered in these methods can be selected from the group consisting of brown rice, oat groats, and combinations thereof. A vegetable source to be formulated in a composition administered in these methods can be selected from the group consisting of beet pulp, tomato pomace, broccoli powder, and combinations thereof.

Age-related conditions prevented and/or treated according to method of the present disclosure include, without limitation, body weight increase, increased adiposity, decreased lean body mass, oxidative stress, oxidative damage, decreased renal function, increased blood levels of microbial toxins, excessive inflammatory response, or a combination of two or more thereof.

In certain embodiments, practice of the methods of prevention and/or treatment described herein result in at least one improvement from among decreased adiposity, increased lean body mass, decreased p-cresol blood levels, decreased 3-indoxyl sulfate blood levels, increased L-carnitine blood levels, decreased oxidized glutathione blood levels, decreased 8-hydroxyguanosine blood levels, increased glomerular filtration, decreased creatinine blood levels, or a combination of two or more thereof in the treated animal.

In certain embodiments, therefore, practice of the methods of prevention and/or treatment described herein result in decreased adiposity, increased lean body mass, enhanced kidney health, reduced inflammation, reduced oxidative stress, and improved digestive health. In particular aspects of these embodiments practice of the methods of prevention and/or treatment described herein result in maintained body decreased adiposity, increased lean body mass, increased glomerular filtration rate, decreased levels of circulating creatinine, decreased levels of circulating 8-hydroxyguanosine, increased levels of circulating L-carnitine, decreased levels of circulating oxidized glutathione, decreased levels of circulating p-cresol sulfate, decreased levels of circulating 3-inoxyl sulfate, or combinations of two or more thereof.

The animal treated according to the methods described herein is an animal in need of such treatment. In certain embodiments, that animal is a companion animal, *e.g.,* a house pet (dog, cat, rabbit *etc*.). In one embodiment, the animal is a domesticated companion animal or "house" pet, such as canine or a feline. In one aspect, the animal is a dog. In another aspect the animal is a cat.

### Examples

### Example 1: Composition of Administered Foods

The foods administered to the felines in the Examples below include a Pre-feed composition provided to the animals before initiation of the studies, as well as a Control Food and an illustrative Anti-Aging Food of the invention administered in the studies. The levels of moisture, ash, protein, crude fat, fiber, and total fatty acids in these foods are provided in Table 1, below.

**Table 1**

| Ingredient | Pre-Feed | Control Food | Anti-Aging Food |
|---|---|---|---|
| Moisture | 6.5 % | 6.8% | 6.3% |
| Ash | 4.9% | 5.2% | 6.2% |
| Crude Fat | 21.1% | 20.8% | 15.3% |
| Crude Fiber | 1.8% | 3.0% | 2.2% |
| Protein | 33.4% | 32.6% | 31.8% |
| Total Fatty Acids | 18.9% | 18.5% | 13.7% |
| Carbohydrate* | 32.2% | 31.7% | 38.2% |

| | | | |
|---|---|---|---|
| * Carbohydrate (Nitrogen-free extract) ≡ 100% - (% Protein + % Fat % Ash + % Fiber + % Moisture) | | | |

The Anti-Aging Food was formulated with protein sources that included chicken, "pea protein concentrate," and corn gluten meal, carbohydrate sources including brown rice, and oat groats, and vegetable sources including beet pulp, tomato pomace, and broccoli powder. Although similar in overall composition, the Control Food did not include the combination of chicken, "pea protein concentrate," corn gluten meal, brown rice, oat groats, beet pulp, tomato pomace, and broccoli powder, much less each within the concentrations described herein. That is, although the Control and Anti-Aging Foods are both formulated to meet the nutritional requirements of the felines to be fed those compositions, the sources of ingredients used to formulate those diets differ from one another.

Administration of the Anti-Aging Food of Table 1 resulted in an improvement in a series of biochemical markers as set for the in the following Examples and Tables. These results demonstrated that the methods described herein would be useful for the prevention and/or treatment of age related conditions in an animal in need thereof, particularly in a companion animal, *e.g.,* a cat or a dog.

### Example 2: Improved Body Condition

An age-related condition of animals, e.g., cats and dogs, is an increase in body fat, often accompanied by a decrease in body lean mass (muscle and bone) as well as increase in total body weight. As demonstrated in this Example, feeding cats the Anti-Aging Food of Table 1 resulted in maintenance of their body weight. Over the 180-day study, there was little change in the body weight of the cats fed either the Anti-Aging Food of Table 1 as compared to the Control Food, *i.e.,* the weight variation of animals fed the Control Food at the 90 and 180-day time points were, respectively, approximately 0.6% below and 0.6% above the average initial weight of the cats, while the weight variation of cats fed the Anti-Aging Food at the 90 and 180-day time points were, respectively, 1.8% below and 1.1% below the average initial weight of the cats.

However, feeding cats the Anti-Aging Food of Table 1 led to both a decrease in body fat and an increase in non-fat body mass (lean body mass), on an absolute basis (as compared to initial data) as well as on a relative basis (as compared to cats fed the Control Food). These data are presented in Table 2 and Table 3, below.

**Table 2**

| Average Grams of Body Fat | | | |
|---|---|---|---|
| Diet Provided | Day 0 | Day 90 | Day 180 |
| Control Food | 875 | 885 | 873 |
| Anti-Aging Food | 876 | 835 | 759 |

The data of Table 2 demonstrate that feeding the Anti-Aging Food of Table 1 to cats led to a decrease in total body fat of the animal, compared to both the initial body fat levels of the Test cat population as well as to the Control population of cats.

One basis for the benefits provided by the Anti-Aging Food of Table 1 may be provided by an increase in the circulating concentration of L-carnitine. When the same amount of L-carnitine was included in the food, it would have been expected that the circulating level of L-carnitine would be constant. However, as demonstrated by the data of Table 3, that was not observed. Rather there was a demonstrated increase in circulating levels of L-carnitine in the treated population.

**Table 3**

| Circulating L-Carnitine Levels | | | |
|---|---|---|---|
| Diet Provided | Day 0 | Day 45 | Day 90 |
| Control Food | 1 | 0.69 | 0.67 |
| Anti-Aging Food | 1 | 1.61 | 1.67 |

The data of Table 3 are presented as ratio of the L-carnitine blood level at the indicated time point to the L-carnitine measured at the beginning of the study. The data of Table 4 demonstrate that after 180 days of feeding the test cats the Anti-Aging Food of Table 1 resulted in an increased circulating levels of L-carnitine in those cats as compared to the levels observed in the control population of cats fed the Control Food.

Accordingly, the above data demonstrate that feeding an Anti-Aging Food of the invention will prevent and/or treat age-related conditions of companion animals, here cats, in need thereof, including age-related increases in body fat, age-related decreases in body lean mass (muscle and bone), as well as age-related increases in total body weight.

### Example 3: Improved Renal Function

An age-related condition of animals, e.g., cats and dogs, is a decrease in kidney function. As demonstrated in this Example, feeding cats the Anti-Aging Food of Table 1 resulted in improved renal function, both with respect to an increase in glomerular filtration rate as well as with respect to a decrease in circulating creatinine levels. The data for the former effect are presented in Table 4.

**Table 4**

| Glomerular Filtration Rate (mL/min/Kg) | | | |
|---|---|---|---|
| Diet Provided | Day 0 | Day 90 | Day 180 |
| Control Food | 1.9 | 2.07 | 2.08 |
| Anti-Aging Food | 1.9 | 2.49 | 2.36 |

These data establish that, over the 180-day study, there was substantial increase in glomerular filtration rate in cats fed the Anti-Aging Food of Table 1, as compared to that of the cats fed the Control Food.

Data demonstrating the decrease in circulating creatinine levels resulting from administration of the Anti-Aging Food of Table 1 are provided in Table 5.

**Table 5**

| Circulating Creatinine Levels (mg/dL) | | | |
|---|---|---|---|
| Diet Provided | Day 0 | Day 90 | Day 180 |
| Control Food | 1.23 | 1.14 | 1.24 |
| Anti-Aging Food | 1.23 | 0.99 | 0.99 |

These data establish that, over the 180-day study, there was a substantial decrease in circulating creatinine levels in the cats fed the Anti-Aging Food of Table 1, as compared to that of the cats fed the Control Food.

Accordingly, the above data demonstrate that feeding a composition of the invention will prevent and/or treat an age-related condition of companion animals, e.g., cats and dogs, the condition being a decrease in kidney function.

### Example 4: Decreased Circulating Levels of Toxic Metabolites

Another age-related condition of animals, e.g., cats and dogs, is an increase in the circulating levels of bacterially-produced toxic materials. Two illustrative examples of which are p-cresol *(e.g.,* as p-cresol sulfate) and 3-hydroxy indole (as 3-indoxyl sulfate).

The p-cresol and 3-hydroxy indole levels were determined by a commercial laboratory (Metabolon, Inc, Durham, NC). Serum samples were solvent-extracted and analyzed by mass spectroscopy and either gas chromatography (hydrophobic molecules), or liquid chromatography (hydrophilic molecules). Data for the metabolites of interest (here p-cresol and 3-hydroxy indole) were normalized by calculating the median values for each run-day block ("block normalization"), to minimize any potential inter-day drift in instrument gain, without interfering with intra-day sample variability.

As demonstrated in this Example, feeding cats the Anti-Aging Food of Table 1 resulted in lower circulating levels of both of these toxic materials. The data obtained are presented in Table 6.

**Table 6**

| Circulating Toxic Metabolite Levels | | | | |
|---|---|---|---|---|
| Metabolite | Day 0 | Day 45 | Day 90 | Day 180 |
| p-Cresol Sulfate | 1.04 | 0.82 | 0.54 | 0.81 |
| 3-Indoxyl sulfate | 1.39 | 0.52 | 0.5 | 0.5 |

The data of Table 6 are presented as the ratio of the concentration of circulating metabolite in the population of cats fed the Anti-Aging Food of Table 1 to the concentration of circulating metabolite in the population of cats fed the Control Food. These data establish that, over the 180-day study, there was a substantial decrease in the levels of both of these bacterially-produced, toxic metabolites.

Accordingly, the above data demonstrate that feeding a composition of the invention will prevent and/or treat an age-related condition of companion animals, e.g., cats and dogs, the condition being increased levels of bacterially-produced, toxic metabolites such as but not limited to p-cresol sulfate and 3-hydroxy indole.

### Example 5: Improved Oxidation Status

A further age-related condition of animals, e.g., cats and dogs, is oxidative damage, e.g., oxidative damage to the DNA of the afflicted animal. As demonstrated herein, administration of a composition of the invention, e.g., the Anti-Aging Food of Table 1, ameliorates or prevents such oxidative damage, improving the oxidative status of the treated cats, as illustrated both by the reduction in urine levels of 8-hydroxydeoxyguanosine (a biomarker of oxidative damage to DNA), as well as by the reduction of the circulating levels of oxidized glutathione.

The reduction in urine levels of 8-hydroxydeoxyguanosine is demonstrated by the data presented in Table 7.

**Table 7**

| 8-Hydroxydeoxyguanosine Levels in Urine | | | | |
|---|---|---|---|---|
| Diet Provided | Day 0 | Day 45 | Day 90 | Day 180 |
| Control Food | 0 | 0.95 | 7.28 | 7.92 |
| Anti-Aging Food | 0 | -7.01 | -7.4 | -2.21 |

The data of Table 7 are presented as the numeric change in the ratio of 8-hydroxydeoxyguanosine (in ng) to the level of creatinine (mg) in urine. These data establish that, over the 180-day study, there was a substantial decrease in the levels of 8-Hydroxydeoxguanosine in the urine of cats fed the Anti-Aging Food of Table 1 as compared to the levels of 8-Hydroxydeoxguanosine measure in the cats fed the Control Food.

The reduction in circulating levels of oxidized glutathione is demonstrated by the data presented in Table 8.

**Table 8**

| Circulating Levels of Oxidized Glutathione | | | | |
|---|---|---|---|---|
| | Day 0 | Day 45 | Day 90 | Day 180 |
| Oxidized Glutathione | 0.94 | 0.63 | 0.54 | 0.54 |

The data of Table 8 are presented as the ratio of the amount of circulating oxidized glutathione measured in the cats fed the Anti-Aging Food of Table 1 to the amount of circulating oxidized glutathione measured in the control cats fed the Control Food. These lower levels of oxidized are indicative of an improvement in oxidative status of the treated cats as compared to the control cats and therefore indicative of improved resistance to oxidative damage.

Accordingly, administration of a composition of the invention is useful as a method for prevention and/or treatment of age-related conditions involving a decrease in the oxidative status of the aging animal and/or oxidative damage to that aging animal.

The collective data provide above demonstrate that administration of a composition for use according to the invention would be useful for the prevention and/or treatment of an age related condition in a companion animal, e.g., a cat or a
dog, in need thereof, including conditions involving increased oxidative damage, poor gastrointestinal health, declining renal function, a body composition with elevated body fat and diminished lean body mass, or a combination thereof.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. All percentages expressed herein are by weight of the composition on dry matter basis unless specifically stated otherwise.

## Claims

1. A composition suitable for an animal in the composition comprising a protein source, a carbohydrate source, and a vegetable source;
wherein the protein source is a combination of chicken, pea protein concentrate, and corn gluten meal;
wherein the carbohydrate source is a combination of brown rice and oat groats; and
wherein the vegetable source is a combination of beet pulp, tomato pomace, and broccoli powder.

2. The composition of claim 1 comprising chicken in an amount from 5% to 25% based on the total weight of the composition on a dry matter basis, or comprising pea protein concentrate in an amount from 10% to 45% based on the total weight of the composition on a dry matter basis, or comprising corn gluten meal in an amount from 10% to 45% based on the total weight of the composition on a dry matter basis.

3. The composition of claim 1 comprising a protein source in an amount from 5% to 45% based on the total weight of the composition on a dry matter basis.

4. The composition of claim 1 comprising brown rice in an amount from 8% to 40% based on the total weight of the composition on a dry matter basis, or comprising oat groats in an amount from 8% to 40% based on the total weight of the composition on a dry matter basis.

5. The composition of claim 1 wherein the carbohydrate source is present in an amount from 8% to 40% based on the total weight of the composition on a dry matter basis.

6. The composition of claim 1 comprising beet pulp in an amount from 1,9% to 10% beet pulp based on the total weight of the composition on a dry matter basis, or comprising tomato pomace in an amount from 1,9% to 10% tomato pomace based on the total weight of the composition on a dry matter basis, or comprising broccoli powder in an amount from 1, 9% to 10% by dry weight of based on the total weight of the composition on a dry matter basis.

7. The composition of claim 1 wherein the vegetable source is present in an amount from 1,9% to 10% based on the total weight of the composition on a dry matter basis.

8. The composition of claim 1, wherein (a) the composition is a nutritionally complete diet for an adult companion animal, optionally wherein the adult companion animal is a canine or a feline; or (b) the composition is a nutritionally complete diet for an aged companion animal, optionally wherein the aged companion animal is a canine or a feline.

9. The composition of any preceding claim, for use in treating or preventing a health affecting age-related condition in an animal in need thereof, wherein the composition is to be administered to the animal in an effective amount;
wherein the age-related condition is one of body weight increase, increased adiposity, decreased lean body mass, oxidative stress, decreased renal function, increased blood levels of microbial toxins, or a combination of two or more thereof,
wherein the animal is a companion animal and
wherein the companion animal is an aged feline.

## Patentansprüche

1. Eine für ein Tier geeignete Zusammensetzung, wobei die Zusammensetzung umfasst:
eine Proteinquelle, eine Kohlenhydratquelle und eine pflanzliche Quelle;
wobei die Proteinquelle eine Kombination von Hähnchen, Erbsenproteinkonzentrat und Maisglutenmehl ist;
wobei die Kohlenhydratquelle eine Kombination von Naturreis und Hafergrütze ist; und
wobei die pflanzliche Quelle eine Kombination von Rübenpulpe, gepresster Tomatenfruchtmasse und Brokkolipulver ist.

2. Die Zusammensetzung nach Anspruch 1, umfassend Hähnchen in einer Menge von 5% bis 25% bezogen auf das Gesamtgewicht der Zusammensetzung auf einer Trockenmaterialbasis oder umfassend Erbsenproteinkonzentrat in einer Menge von 10% bis 45% bezogen auf das Gesamtgewicht der Zusammensetzung auf einer Trockenmaterialbasis oder umfassend Maisglutenmehl in einer Menge von 10% bis 45% bezogen auf das Gesamtgewicht der Zusammensetzung auf einer Trockenmaterialbasis.

3. Die Zusammensetzung nach Anspruch 1, umfassend eine Proteinquelle, die in einer Menge von 5% bis 45% bezogen auf das Gesamtgewicht der Zusammensetzung auf einer Trockenmaterialbasis vorliegt.

4. Die Zusammensetzung nach Anspruch 1, umfassend Naturreis in einer Menge von 8% bis 40% bezogen auf das Gesamtgewicht der Zusammensetzung auf einer Trockenmaterialbasis oder umfassend Hafergrütze in einer Menge von 8% bis 40% bezogen auf das Gesamtgewicht der Zusammensetzung auf einer Trockenmaterialbasis.

5. Die Zusammensetzung nach Anspruch 1, wobei die Kohlenhydratquelle in einer Menge von 8% bis 40% bezogen auf das Gesamtgewicht der Zusammensetzung auf einer Trockenmaterialbasis vorliegt.

6. Die Zusammensetzung nach Anspruch 1, umfassend Rübenpulpe in einer Menge von 1,9% bis 10% Rübenpulpe bezogen auf das Gesamtgewicht der Zusammensetzung auf einer Trockenmaterialbasis oder umfassend gepresste Tomatenfruchtmasse in einer Menge von 1,9% bis 10% bezogen auf das Gesamtgewicht der Zusammensetzung auf einer Trockenmaterialbasis oder umfassend Brokkolipulver von 1,9% bis 10% bezogen auf das Gesamtgewicht der Zusammensetzung auf einer Trockenmaterialbasis.

7. Die Zusammensetzung nach Anspruch 1, wobei die pflanzliche Quelle in einer Menge von 1,9% bis 10% bezogen auf das Gesamtgewicht der Zusammensetzung auf einer Trockenmaterialbasis vorliegt.

8. Die Zusammensetzung nach Anspruch 1, wobei (a) die Zusammensetzung eine was die Ernährung betrifft vollständige Nahrung für ein ausgewachsenes Haustier ist, wobei wahlweise das ausgewachsene Haustier ein Hund oder eine Katze ist; oder (b) die Zusammensetzung eine was die Ernährung betrifft vollständige Nahrung für ein betagtes Haustier ist, wobei wahlweise das betagte Haustier ein Hund oder eine Katze ist.

9. Die Zusammensetzung nach irgendeinem vorhergehenden Anspruch zur Verwendung bei der Behandlung oder Prävention eines gesundheitsbeeinträchtigenden mit dem Alter zusammenhängenden Zustands bei einem Tier, das dessen bedarf, wobei die Zusammensetzung dem Tier in einer wirksamen Menge verabreicht werden muss;
wobei der mit dem Alter zusammenhängende Zustand einer von Körpergewichtszunahme, erhöhter Adipositas, verringerter fettfreier Körpermasse, oxidativem Stress, verminderter Nierenfunktion, erhöhten Blutspiegeln von mikrobiellen Toxinen ist, oder eine Kombination von zwei oder mehreren davon
wobei das Tier ein Haustier ist und
wobei das Haustier eine betagte Katze ist.

## Revendications

1. Composition appropriée pour un animal, la composition comprenant
une source de protéines, une source de glucides et une source de légumes ;
dans laquelle la source de protéines est une combinaison de poulet, de concentré de protéines de pois, et de farine de gluten de maïs ;
dans laquelle la source de glucides est une combinaison de riz brun et de gruau d'avoine ; et
dans laquelle la source de légumes est une combinaison de pulpe de betterave, de pulpe de tomate et de poudre de brocoli.

2. Composition selon la revendication 1, comprenant du poulet en une quantité de 5 % à 25 % par rapport au poids total de la composition sur une base de matière sèche, ou comprenant un concentré de protéines de pois en une quantité de 10 % à 45 % par rapport au poids total de la composition sur une base de matière sèche, ou comprenant de la farine de gluten de maïs en une quantité de 10 % à 45 % par rapport au poids total de la composition sur une base de matière sèche.

3. Composition selon la revendication 1, comprenant une source de protéine présente en une quantité de 5 % à 45 % par rapport au poids total de la composition sur une base de matière sèche.

4. Composition selon la revendication 1, comprenant du riz brun en une quantité de 8 % à 40 % par rapport au poids total de la composition sur une base de matière sèche, ou comprenant du gruau d'avoine en une quantité de 8 % à 40 % par rapport au poids total de la composition sur une base de matière sèche.

5. Composition selon la revendication 1, dans laquelle la source de glucides est présente en une quantité de 8 % à 40 % par rapport au poids total de la composition sur une base de matière sèche.

6. Composition selon la revendication 1, comprenant de la pulpe de betterave en une quantité de 1,9 % à 10 % de pulpe de betterave par rapport au poids total de la composition sur une base de matière sèche, ou comprenant de la pulpe de tomate en une quantité de 1,9 % à 10 % par rapport au poids total de la composition sur une base de matière sèche, ou comprenant de la poudre de brocoli de 1,9 % à 10 % par rapport au poids total de la composition sur une base de matière sèche.

7. Composition selon la revendication 1, dans laquelle la source de légumes est présente en une quantité de 1,9 % à 10 % par rapport au poids total de la composition sur une base de matière sèche.

8. Composition selon la revendication 1, dans laquelle (a) la composition est un régime nutritionnellement complet pour un animal de compagnie adulte, éventuellement dans laquelle l'animal de compagnie adulte est un canin ou un félin ; ou (b) la composition est un régime nutritionnellement complet pour un animal de compagnie âgé, éventuellement dans laquelle l'animal de compagnie âgé est un canin ou un félin.

9. Composition selon l'une quelconque des revendications précédentes, pour une utilisation dans le traitement ou la prévention d'une affection liée à l'âge affectant la santé chez un animal en ayant besoin, dans laquelle la composition doit être administrée à l'animal en une quantité efficace ;
dans laquelle l'affection liée à l'âge est une affection parmi l'augmentation de poids corporel, une adiposité accrue, la diminution de la masse corporelle maigre, le stress oxydatif, la diminution de la fonction rénale, l'augmentation des taux sanguins de toxines microbiennes, on une combinaison de deux ou plusieurs de celles-ci,
dans laquelle l'animal est un animal de compagnie, et
dans laquelle l'animal de compagnie est un félin âgé.
